# EUROPEAN PATENT SPECIFICATION

(11) **EP 1 599 391 B1**
(45) Date of publication and mention of the grant of the patent: **12.12.2007**
(21) Application number: 04709395.0
(22) Date of filing: 09.02.2004
(51) Int. Cl.: B65D 75/58, B65D 85/16

(54) **A FOLDED ABSORBENT ARTICLE PACKED IN A PACKAGING WRAPPER**
GEFALTETER SAUGFÄHIGER GEGENSTAND, DER IN EINER VERPACKUNGSHÜLLE VERPACKT IST
ARTICLE ABSORBANT PLIE EMBALLE DANS UNE ENVELOPPEUSE

(30) Priority: 10.02.2003 SE 0300346
(43) Date of publication of application: 30.11.2005
(73) Proprietor: SCA Hygiene Products AB, 405 03 Göteborg (SE)
(72) Inventor: BOGREN, Maria, 436 39 Askim (SE); HERMANSSON, Sofia, 426 71 Västra Frölunda (SE)
(74) Representative: Andersson, Per Rune
(86) International application number: PCT/SE2004/000161
(87) International publication number: WO 2004/069684

(56) References cited:
- US-A- 5 454 601
- US-A1- 2002 060 167
- US-A1- 2002 063 076
- US-B1- 6 309 104

## Description

### TECHNICAL FIELD

The present invention concerns a folded absorbent article for absorption of body fluids and being packaged in a packaging wrapper, wherein the packaging wrapper comprises a container part having an inside and an outside and an opening for removal of the absorbent article, and a lid part which has an open position and a closed position, wherein the lid part in the closed position closes the opening of the container part and is releasably attached to the outside of the container part.

### BACKGROUND ART

Absorbent articles for personal hygiene and particularly those articles which are intended to be worn inside ordinary underpants and which are applied in the underpants by the user are often packaged individually in a packaging wrapper see US 2002/0063076 A. Thereby, it is easy for the user to carry along only a few absorbent articles in a simple and hygienic manner, for example in a handbag. Some examples of the kind of absorbent articles which is concerned are sanitary napkins, pantyliners and incontinence shields for persons with mild to moderate incontinence.

The most usual way of single-wrapping absorbent articles is to fold them into a smaller size and to wrap them in a thin packaging wrapper of plastic or paper. Such a packaging wrapper often consists of a rectangular piece of material which has been folded twice in its transverse direction and subsequently joined along its side edges so that a pouch-like container for the absorbent article has been formed, and a lid part which can close the container. The lid part is often attached with releasable joins such as tearable welds or releasable adhesive to the edge joins on the outside of the container part when the packaging wrapper is in the closed position. Additional sealing between the lid part and the container part can also be used along the end edge of the lid part. Such sealing can, for instance, be of releasable adhesive or in the form of hook-and-loop surfaces. If the sealing member permits resealing, it can also be used to close the packaging wrapper if it is used to wrap an absorbent article after use.

However, it has become apparent that the known single-wrap packages may be difficult to open. Since the packaging material is thin and flexible, the lid part will cling closely to the outside of the container part. This problem is accentuated by the fact that absorbent articles of the kind concerned here are packaged with a plurality of individually wrapped articles in a larger outer package. Thereby, the articles are rather heavily compressed which means that the packaging wrappers are also pressed together which, naturally, means that the lid part of the packaging wrapper is pressed against the outside of the container part. Due to the strong compression of the packaging material and since the packaging material is so thin and flexible, it has been discovered that many users find it difficult to grip the edge of the lid part so that the packaging wrapper can be opened. It can be very hard both to distinguish the edge visually and to feel its presence with the fingers. Obviously, this is a particular problem for persons having reduced eyesight or having limited dexterity. Since the occurrence of incontinence increases with increasing age, like poor eyesight and problems with poor hand mobility, handling of the known single-wrap packages is a common and very irritating problem for older users of absorbent articles.

Consequently, one object of the present invention is to offer a single-wrapped absorbent article in a wrapper which is easier to open than previously known packaging wrappers.

### DISCLOSURE OF INVENTION

Hence, in accordance with the invention a folded absorbent article according to claim 1 has been produced.

As has previously been mentioned, it can be difficult to open a single-wrap package with an absorbent article since the packaging material is often thin and it is difficult to get a grip on the lid part of a packaging wrapper. By arranging a distance element between the lid part and the container part on the packaging wrapper, it is made easier for the user to find the edge of the lid part and to get the fingers under the edge and lift up the lid part from the container part.

In accordance with one embodiment of the invention the lid part has a first end at the opening of the container part and a second end with an end edge, wherein the distance element is placed by the end edge of the lid part. This embodiment is advantageous since it ascertains that the end edge of the lid part is lifted up from the container part within at least a portion of the edge.

In accordance with another embodiment of the invention the packaging wrapper is formed from a rectangular piece of material having a length-direction and a cross-direction and having two side edges extending in the length-direction and two end edges extending in the cross-direction. Further, the packaging wrapper has two fold lines arranged in the cross-direction, said fold lines dividing the packaging wrapper into a first end panel, a second end panel and a central panel. The second end panel and the central panel are joined to each other in side edge joins along the side edges and thereby form the container part of the packaging wrapper. The first end panel forms the lid part of the packaging wrapper. In accordance with the invention, the side edge joins may be formed in any suitable matter. Common ways of weld joining packaging wrappers is through welding with heat or ultrasound, often simultaneously with embossing of the join. It is also common to treat the joined surfaces with an agent to increase their openability. Moreover, it is possible to make joins and sealing members with the aid of adhesive.

In accordance with the invention the lid part is advantageously attached to the side edge joins with a tearable attachment. Such a tearable attachment may, for instance, consist of a tearable weld join.

In order to obtain good grippability of the lid part, it is suitable that the distance element has the ability of creating a distance of at least 0.5 mm between the lid part and the container part.

A number of different types of distance elements may be used within the scope of the invention. Accordingly, the distance element may comprise a resiliently compressible material, an open cell foam material, a fibrous wadding, a helical spring, a plate spring, or an elongated elastic element which is attached with pre-stretching on the lid part. Combinations of different types of distance elements are also possible.

The distance element is suitably designed so that it has a first inactive state and a second active state. Such a design is favourable since it permits the single-wrapped absorbent article to be packaged in an outer package without the distance element taking up space in the outer package. For articles of the disposable kind, i.e. absorbent articles that are intended to be discarded after a single use, it is particularly important that they take up as little space as possible during transport and storage before use. For this reason, it is advantageous if the distance element can be compressed to a small volume or can assume a generally planar shape when the article is packaged together with other articles in an outer package.

The distance element can suitably be brought from the inactive state to the active state either by automatically assuming the active state for instance when the single-wrapped article is taken out of the outer package or by manipulation of the distance element.

Some examples of distance elements of the kind first mentioned are compressed waddings and foam materials and helical springs which rise or expand by themselves when they are no longer compressed inside an outer package. Another example of this type of distance element is elastic elements, which can be kept stretched when the single-wrapped articles are tightly packed in an outer package but constrict when the single-wrapped articles are taken out of the outer package. In addition, the activation of the distance elements will in these cases to a lesser or greater extent take place as the outer package is being emptied and the compression of the single-wrapped articles is diminished.

Examples of distance elements of the last mentioned kind are plate springs or similar, which can be made to curve from an initially flat state.

In order to ascertain that the packaging wrapper is well sealed before use, it is advantageous if the distance element comprises a releasable sealing member for the lid part of the packaging wrapper. Such a sealing member is preferably a resealable sealing member, whereby it can be used for sealing the packaging wrapper when it is being used as a protective wrapper for a used absorbent article. It is common to use adhesive areas, tape tabs, or the like as sealing members. However, it is also possible to use mechanical sealing members such as hook-and-loop surfaces press studs, or similar.

### BRIEF DESCRIPTION OF DRAWINGS

In the following, the invention will be described more closely with reference to the figures which are shown in the appended drawings:
- Fig. 1: a packaging wrapper enclosing a packaged sanitary napkin;
- Fig. 2: a section taken along the line 11-11 through the packaging wrapper in Fig. 1 ;
- Fig. 3: a planar view of the packaged sanitary napkin in Figs. 1 and 2, seen from the side which is intended to be facing the user during use;
- Fig. 4: a packaging wrapper having a distance element in the form of an elastic band;
- Fig. 5: a section taken along the line V-V through the distance element in Fig. 4;
- Fig. 6: a packaging wrapper having a distance element in the form of a plate spring;
- Fig. 7: a section taken along the line VII-VII through the distance element in Fig. 6;
- Fig. 8: a packaging wrapper having a first distance element in the form of a helical spring and a second distance element in the form of a band of wadding;
- Fig. 9: a section taken along the line IX-IX through the first distance element in Fig. 8; and
- Fig. 10: a section taken along the line X-X through the second distance element in Fig. 8.

### DESCRIPTION OF EMBODIMENTS

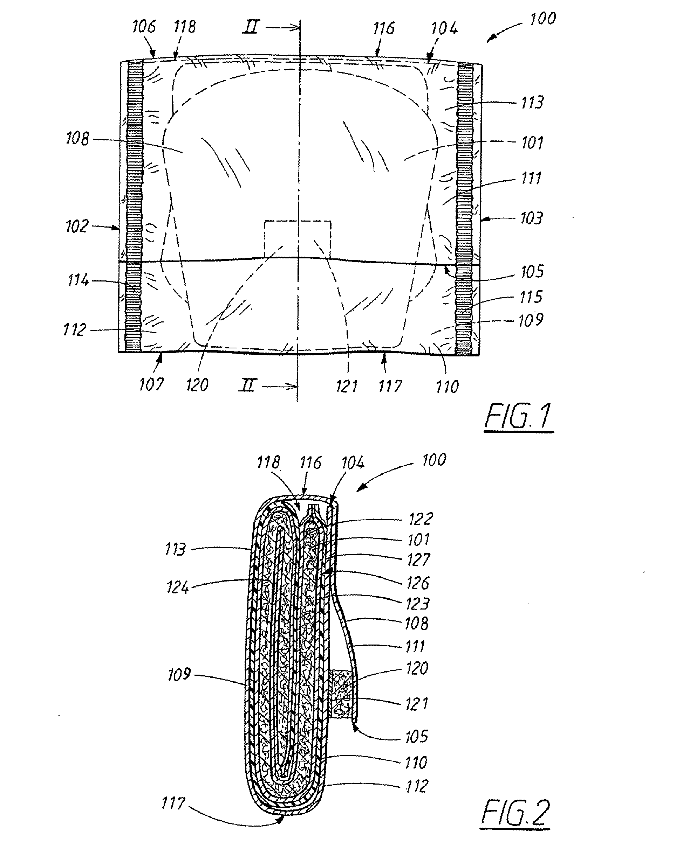

Figs. 1 and 2 show a packaging wrapper 100 having a sanitary napkin 101 packaged therein. The packaging wrapper 100 is formed from a piece of material having rectangular shape wherein the piece of material 113 has two side edges 102, 103 which also form side edges 102,103 on the packaging wrapper, and two end edges 104,105.

Suitable materials for a packaging wrapper in accordance with the invention are thin plastic films, for example polyolefin films such as polyethylene films or polypropylene films. Naturally, it is also possible to use paper or nonwoven material. If the packaging wrapper shall also serve as a release material for an adhesive fastening device on the sanitary napkin 101, it is usually suitable that the surface of the packaging wrapper which comes into contact with the fastening device has some kind of release treatment. A common release treatment is siliconisation.

The packaging wrapper is folded along a first fold line 106 and a second fold line 107 so that three panels 108,109,110 are formed, wherein a first end panel 108 constitutes the lid part 111 of the packaging wrapper and a second end panel 110 and the central panel 109, which is situated between the end panels, together form the container part 112 of the packaging wrapper. The container part 112 is closed along the side edges 102,103 of the packaging wrapper 100 by edge joins 114,115. In the shown example, the edge joins are embossed welds but it is naturally possible to make edge joins in other ways, for instance with adhesive. The lid part 111 is also attached in the edge joins, implying that the packaging wrapper as shown in Figs. 1 and 2 is in a closed position. It is advantageous if the lid part 111 can be readily detached from the container part along the edge joins 114, 115 without tearing of the packaging material. This can be accomplished by making the edge joins 114, 115 openable at least between the lid part 111 and the container part 112. For some embodiments, it may be suitable that the edge joins 114, 115 are completely openable, for instance if it is desirable to allow complete unfolding of the packaging wrapper when the sanitary napkin 101 is to be taken out of the packaging wrapper.

As has already been mentioned, the packaging wrapper 100 in its folded and joined state has two side edges 102, 103. Further, the packaging wrapper has a first end edge 116 and a second end edge 117, which extend perpendicularly to the side edges 102,103 and which coincide with the first and second fold line 106,107 of the packaging wrapper. The first end edge 116 also constitutes an inner edge of the lid part while the outer edge 105 of the lid part 111 coincides with one of the end edges 105 of the piece of material 113 and is located some way down on the second end panel 110 on the outside of the container part. In addition, the packaging wrapper 100 has an opening 118 which is located between the second end panel 110 and the central panel 109 at the first end edge 116 of the packaging wrapper inside the first fold line 106 in the piece of material 113.

A distance element 120 in the form of a cuboid piece of foamed plastic is arranged between the lid part 111 and the container part by the outer edge 105 of the lid part 111 and centrally between the side edges 102,103 of the packaging wrapper. The foamed plastic is preferably an open cell foam material, whereby the distance element 120 can be easily compressed, for instance when the packaged sanitary napkin 101 is inserted into an outer package together with several single-wrapped sanitary napkins. A compressible distance element is favourable since it takes up minimal space and thereby does not lead to the single-wrapped sanitary napkins demanding greater room in the outer package.

The distance element 120 is securely fixed to the lid part 111 for instance by glueing or welding and is attached to the container part 112 with an adhesive area 121 that can be detached from the container part 112 so that the packaging wrapper 100 can be opened. The adhesive area 121 on the distance element 120 is not necessary to the invention and can be excluded if desired or be substituted with some other type of fastening means. It is advantageous to choose to provide the distance element with an adhesive area that can be resealed. In that way, it is possible to use the packaging wrapper as a disposal wrapper for a used sanitary napkin.

The sanitary napkin 101 which is packaged in the packaging wrapper may, of course, be of any kind. Obviously, it is not necessarily a sanitary napkin, it being possible to alternatively package other types of absorbent articles such as pantyliners and incontinence shields in the packaging wrapper shown in Figs. 1 and 2. Thus, the exact design of an absorbent article that is packaged in a packaging wrapper in accordance with the invention is not critical to the invention and a number of different variants and designs of absorbent articles are conceivable. However, the invention is primarily applicable to absorbent articles having such shape and size that they can be folded into a handy format and thereby readily and discreetly be kept and transported in, for example, a pocket, a toilet case, or a handbag.

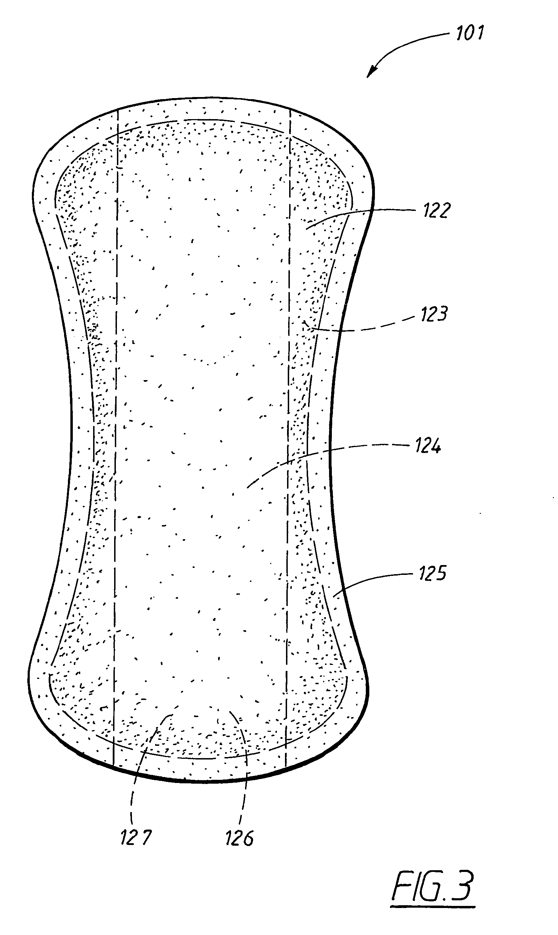

Fig. 3 shows a planar view of the sanitary napkin which is enclosed in the packaging wrapper 100 in Figs. 1 and 2 seen from the side which is intended to be facing the user during use. The sanitary napkin 101 in Fig. 3 is of a conventional construction with a liquid permeable covering layer 122, a liquid impermeable covering layer 123 and an absorption body 124 enclosed between the covering layers 122,123. The covering layers 122,123 are mutually joined to each other for instance by gluing or welding, the join between the covering layers forming a covering edge 125 extending all the way around the periphery of the absorption body 124. Alternative designs of absorbent articles having a liquid barrier layer arranged on the surface of the absorption body which is facing away from the user and inside a liquid permeable outer covering layer which encloses all of the absorption body are also known.

Common liquid permeable covering layers are perforated and non-perforated nonwoven materials, nettings, liquid permeable foam, perforated plastic films, or similar. Combinations and laminates of materials are also useful. Thin plastic film is usually used as a liquid impermeable covering material but it is also known to create a liquid barrier by means of a coating of wax or adhesive. Impermeable foam materials and more or less liquid impermeable nonwoven materials have also been employed.

The absorption body 124 in an absorbent article such as a sanitary napkin or similar is often constructed from one or several layers of absorbent material, such as cellulose fluff pulp, tissue, fibrous wadding, or similar. It is also common to use what is known as superabsorbent materials which are polymers in the form of particles, flakes, fibres, granules or film and which can absorb liquid corresponding to several times their own weight under formation of a liquid containing gel.

In addition, the sanitary napkin in Fig. 3 can comprise components which are not shown in the figures, such as liquid acquisition layers, liquid transport layers, stiffening elements, reinforcement elements, embossings, elastic elements, side barriers, etc. Sanitary napkins and pantyliners which are expected to receive very small amounts of liquid do not have to be equipped with a special absorption body; a surface layer offering a certain amount of absorption capacity may be sufficient.

In order to be able to be attached inside a pair of underpants, the sanitary napkin 101 is provided with a fastening element in the form of an adhesive area 126 being arranged on the liquid impermeable covering layer 123. Before use, the adhesive area 126 is covered by a protective layer 127, for instance of siliconised paper. The adhesive area may be a completely covering coating or may be arranged in a pattern, usually of stripes or dots. Several adhesive areas may be arranged on the liquid impermeable covering layer and may be covered by a common protecting layer or by several separate protecting layers. Naturally, it is possible to eliminate the fastening element or to use other types of fastening elements such as hook-and-loop surfaces, press studs, friction coatings, etc. A particularly common type of combined fastening means and leakage barrier are fastening flaps which are arranged along the side edges of the absorbent article and which in use are folded around the crotch portion of the user's underpants and are attached on the outside of the underpants. Such fastening flaps are often used in combination with fastening adhesive on the underside of the absorbent article.

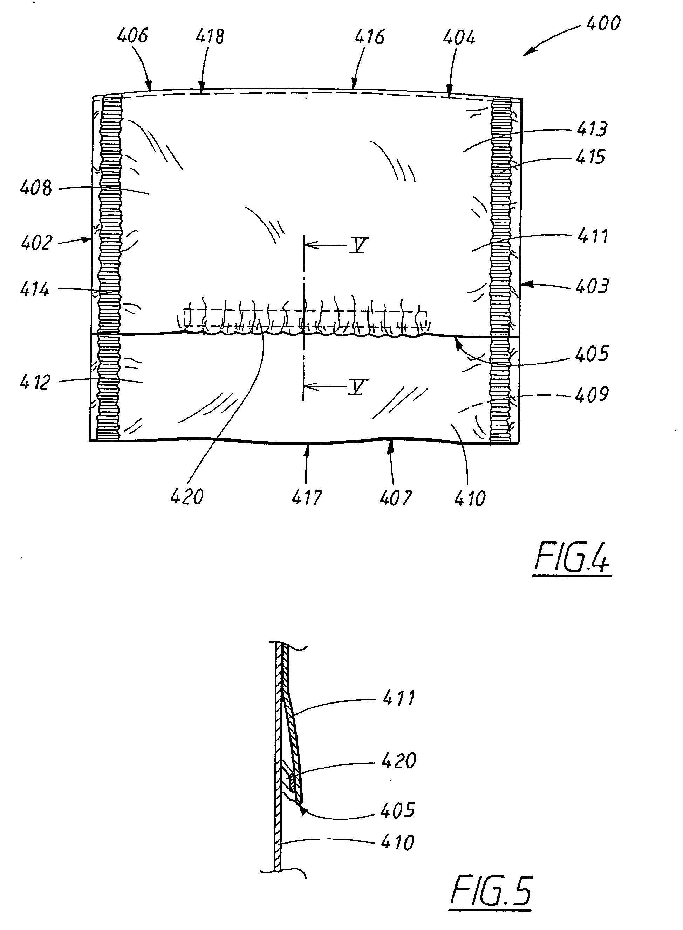

The packaging wrapper 400 which is shown in Fig. 4 differs from the previously described packaging wrapper in that the distance element 420 is constituted by an elastic band which is attached in a pre-stretched state along the outer edge 405 of the lid part 411. Such attachment can, for instance, be accomplished by gluing or welding with heat or ultrasound. It is, of course, not necessary that the distance element 420 has precisely a band shape, elastic netting, threads, or similar also being useful.

The pre-stretched elastic distance element 420 gathers the material closest to the outer edge 405 of the lid part 411 so that it becomes wrinkled and somewhat uplifted from the second end panel 410 of the container part 412. This is shown clearly in Fig. 5. Due to a distance being formed between the lid part 411 and the container part 412, the lid part 411 can be readily gripped and the package opened.

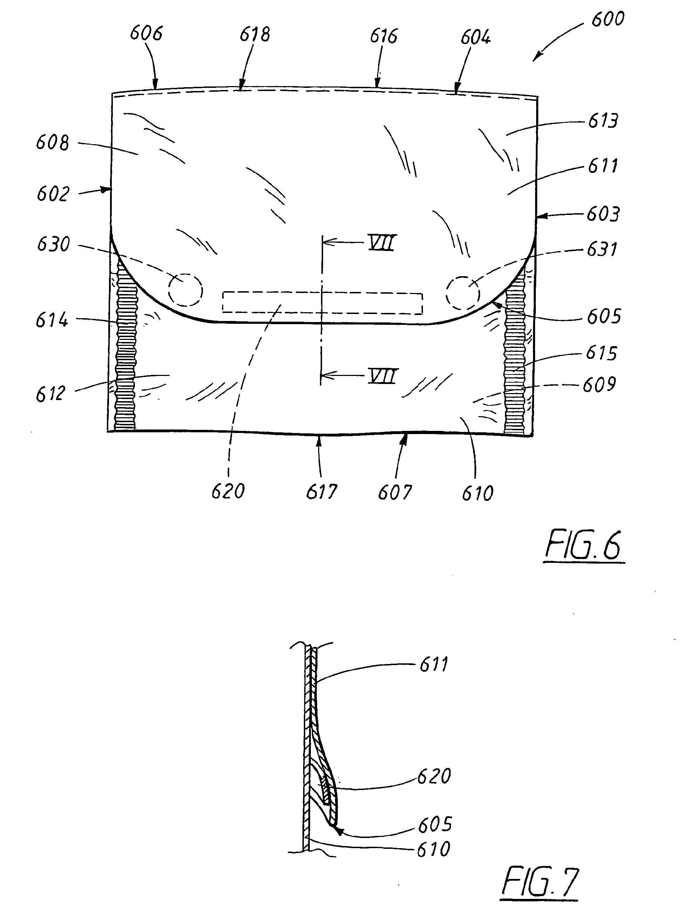

The packaging wrapper 400 shown in Fig. 4 has no seal between the lid part 411 and the container part 412 apart from the attachment which is made in the edge joins 414,415. If a tighter seal is desired, this may for instance be achieved by arranging an openable weld seal or an adhesive area inside the distance element 420, between the distance element 420 and the inner edge 415 of the lid part 411. Such an additional sealing member, but having another placement, is shown in Fig. 6 and is more closely described in the following.

In Fig. 6 is shown a further packaging wrapper 600 having a container part 612 and a lid part 611. The packaging wrapper 600 differs from the previously described packaging wrappers 100,400 in that the lid part 611 is not attached to the container part 612 in the edge joins 614,615 along the side edges 602,603. Instead, sealing members 630,631 are arranged in the form of adhesive areas between the lid part 611 and the container part 612. The sealing members 630,631 are placed close to the outer edge 605 of the lid part 611 on each side of a band shaped distance element 620 and are circular in the shown embodiment. Evidently, the shape, the number and the location of the sealing members may be different from what is shown in Fig. 6. For instance, the circular sealing members 630,631 can be substituted for a band-shaped sealing member. This is then suitably placed inside of the distance element 620, i.e. at a distance from the outer edge 605 of the lid part 611. Instead of or in combination with adhesive sealing members, welds, hook-and-loop closures, or similar may be employed. The sealing members preferably permit that the packaging wrapper is opened without tearing of the sheet of material 613. Further, it is an advantage if the sealing members 630,631 may be used to reseal the packaging wrapper when this is used as a disposal wrapper for a used absorbent article.

The distance element 620 shown in Figs. 6 and 7 consists of a band-shaped plate spring. This can for instance be made of plastic or metal and will curve, preferably when being activated by manipulating or when the packaging wrapper and its contents are being taken out of an outer package, so that the edge 605 of the lid part 611 is lifted up from the end panel 610 of the container part 612 as shown in Fig. 7.

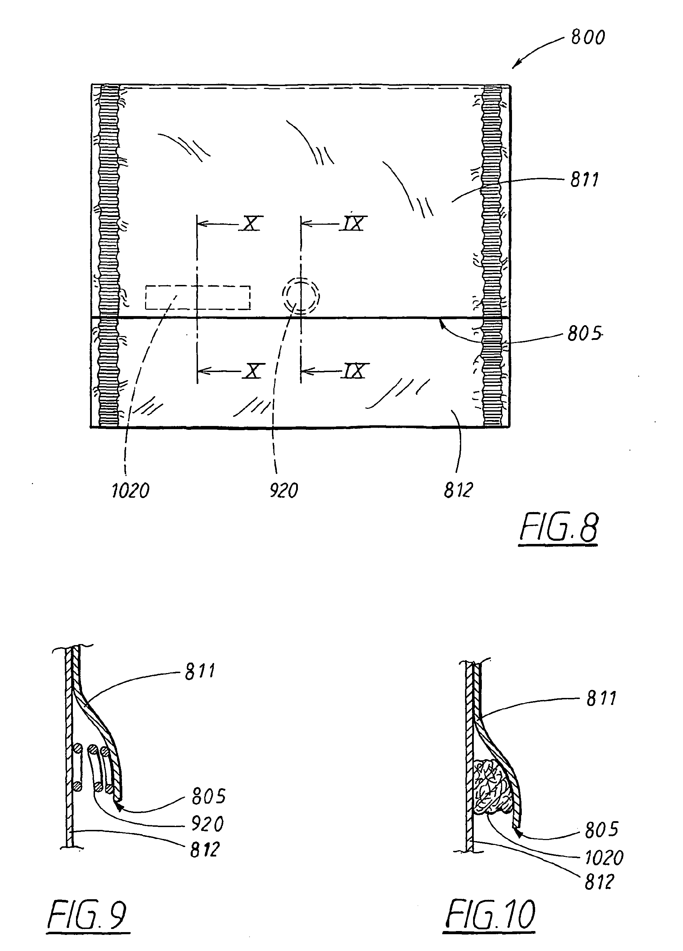

Two further variants of useful distance elements 920, 1020 are shown in Figs. 8-10. Normally, only one type of distance element is used in one and the same packaging wrapper.

The first distance element 920 which is also shown in cross-section in Fig. 9, consists of a helical spring. The helical spring can assume two positions; one being a compressed position where the distance element takes up minimal space and one being a sprung-back position where the distance element 920 lifts up the lid part 811 of the packaging wrapper 800 from the container part 812 so that the lid part can be gripped and the packaging wrapper opened.

The second distance element 1020, which is shown in Figs. 8 and 10, consists of a fibrous, resilient wadding. The second distance element 1020 can also be compressed to a packaging position where it takes up minimal space and can spring back to the distance position shown in Fig. 10 when the compression ceases.

Naturally, the second distance element 1020 can be placed in another way than that shown in Fig. 8. For instance, a distance element in the form of a resilient fibrous wadding can be arranged as a string along all of the outer edge 805 of the lid part.

The invention should not be regarded as being limited by the embodiments described herein. For instance, it is possible within the scope of the invention to package other types of absorbent articles than sanitary napkins in a packaging wrapper in accordance with the invention. The invention is applicable to all kinds of packaging wrappers having a lid part and a container part where it is desirable to improve the grippability of the lid part by creating a distance between the lid part and the container part.

Within the scope of the invention, it is possible to vary the shape and size of the different distance elements which have been disclosed, as well as their placement on the packaging wrapper.

## Claims

1. A folded absorbent article (101) for the absorption of body fluids and being packaged in a packaging wrapper (100), wherein the packaging wrapper (100) comprises a container part (112) having an inside and an outside and an opening (118) for taking out the absorbent article and a lid part (111) having an open position and a closed position, wherein the lid part (111) in the closed position closes the opening (118) of the container part (112) and is releasably attached to the outside of the container part (112), **characterized in that** a distance element (120) is arranged between the lid part (111) and the container part (112), the distance element having the ability of creating a distance of at least 0.5 mm between the lid part and the container part and constituting a means for facilitating opening of the packaging wrapper (100).

2. A folded absorbent article according to claim 1, wherein the lid part (111) has a first end at the opening (118) of the container part and a second end having an end edge (105), wherein the distance element (120) is located by the end edge (105).

3. A folded absorbent article according to claim 1 or 2, wherein the packaging wrapper (100) is formed from a rectangular piece of material (113) having a length-direction and a cross-direction and having two side edges (102,103) extending in the length-direction and two end edges (104, 105) extending in the cross-direction and wherein the packaging wrapper (100) has two fold lines (106,107) arranged in the cross-direction and dividing the packaging wrapper (100) into a first end panel (108), a second end panel (110) and a central panel (109), the second end panel (110) and the central panel (109) being joined to each other in side edge joins (114,115) along the side edges (102,103) thereby forming the container part (112) of the packaging wrapper (100) and that the first end panel (108) forms the lid part (111) of the packaging wrapper (100).

4. A folded absorbent article according to claim 3, wherein the lid part (111) is attached to the side edge joins (114,115) with a tearable attachment.

5. A folded absorbent article according to claim 4, wherein the tearable attachment consists of a tearable weld join.

6. A folded absorbent article according to any one of the preceding claims, wherein the distance element (120) has the ability of creating a distance of at least 0.5 mm between the lid part (111) and the container part (112).

7. A folded absorbent article according to any one of the preceding claims, wherein the distance element (120; 920; 1020) comprises a resiliently compressible material.

8. A folded absorbent article according to any one of the preceding claims, wherein the distance element (120) comprises an open cell foam material.

9. A folded absorbent article according to any one of the preceding claims, wherein the distance element (1020) comprises a fibrous wadding.

10. A folded absorbent article according to any one of the preceding claims, wherein the distance element (920) comprises a helical spring.

11. A folded absorbent article according to any one of the preceding claims, wherein the distance element (620) comprises a plate spring.

12. A folded absorbent article according to any one of the preceding claims, wherein the distance element (120) comprises an elongated elastic element which is attached with pre-stretching on the lid part (411).

13. A folded absorbent article according to any one of the preceding claims, wherein the distance element (120) has a first inactive state and a second active state.

14. A folded absorbent article according to claim 13, wherein the distance element (120) can be brought from the inactive state to the active state by manipulation of the distance element (120).

15. A folded absorbent article according to any one of the preceding claims, wherein the distance element (120) comprises a releasable sealing member (121) for the lid part (111) of the packaging wrapper (100).

16. A folded absorbent article according to claim 15, wherein the sealing member (121) is a resealable sealing member (121).

## Patentansprüche

1. Gefalteter absorbierender Gegenstand (101) zur Absorption von Körpefluiden und verpackt in einer Verpackungshülle (100), wobei die Verpackungshülle (100) einen Aufnahmeabschnitt (112) mit einer Innenseite und einer Außenseite und einer Öffnung (118) zum Herausnehmen des absorbierenden Gegenstands und einen Deckelabschnitt (111) mit einer offenen Position und einer geschlossenen Position umfasst, wobei der Deckelabschnitt (111) in der geschlossenen Position die Öffnung (118) des Aufnahmeabschnitts (112) verschließt und lösbar an der Außenseite des Aufnahmeabschnitts (112) befestigt ist, **dadurch gekennzeichnet, dass** ein Abstandselement (120) zwischen dem Deckelabschnitt (111) und dem Aufnahmeabschnitt (112) angeordnet ist, das die Fähigkeit aufweist einen Abstand von wenigstens 0,5 mm zwischen dem Deckelabschnitt und dem Aufnahmeabschnitt zu erzeugen und eine Einrichtung bildet, um das Öffnen der Verpackungshülle (100) zu erleichtern.

2. Gefalteter absorbierender Gegenstand nach Anspruch 1, bei dem der Deckelabschnitt (111) ein erstes Ende an der Öffnung (118) des Aufnahmeabschnitts und ein zweites Ende mit einer Endkante (105) aufweist, wobei das Abstandselement (120) neben der Endkante (105) angeordnet ist.

3. Gefalteter absorbierender Gegenstand nach Anspruch 1 oder 2, bei dem die Verpackungshülle (100) aus einem rechteckigen Materialstück (113) ausgebildet ist, das eine Längsrichtung und eine Querrichtung sowie zwei Seitenkanten (102, 103), die sich in Längsrichtung erstrecken und zwei Endkanten (104, 105), die sich in Querrichtung erstrecken, aufweist und wobei die Verpackungshülle (100) zwei Faltlinien (106, 107) aufweist, die in Querrichtung angeordnet sind und die Verpackungshülle (100) in ein erstes Endfeld (108), ein zweites Endfeld (110) und ein zentrales Feld (109) unterteilen, wobei das zweite Endfeld (110) und das zentrale Feld (109) an Seitenkantenverbindungen (114, 115) entlang der Seitenkante (102, 103) miteinander verbunden sind, wodurch der Aufnahmeabschnitt (112) der Verpackungshülle (100) gebildet wird und das erste Endfeld (108) den Deckelabschnitt (111) der Verpackungshülle (100) bildet.

4. Gefalteter absorbierender Gegenstand nach Anspruch 3, bei dem der Deckelabschnitt (111) in einer aufreißbaren Befestigung an den Seitenkantenverbindungen (114, 115) angebracht ist.

5. Gefalteter absorbierender Gegenstand nach Anspruch 4, bei dem die aufreißbare Befestigung aus einer aufreißbaren Schweißverbindung besteht.

6. Gefalteter absorbierender Gegenstand nach einem der vorstehenden Ansprüche, bei dem das Abstandselement (120) die Fähigkeit aufweist einen Abstand von wenigstens 0,5 mm zwischen dem Deckelabschnitt (111) und dem Aufnahmeabschnitt (112) zu erzeugen.

7. Gefalteter absorbierender Gegenstand nach einem der vorstehenden Ansprüche, bei dem das Abstandselement (120, 920, 1020) ein elastisch komprimierbares Material umfasst.

8. Gefalteter absorbierender Gegenstand nach einem der vorstehenden Ansprüche, bei dem das Abstandselement (120) einen offenporigen Schaumstoff umfasst.

9. Gefalteter absorbierender Gegenstand nach einem der vorstehenden Ansprüche, bei dem das Abstandselement (1020) eine faserförmige Watte umfasst.

10. Gefalteter absorbierender Gegenstand nach einem der vorstehenden Ansprüche, bei dem das Abstandselement (920) eine Schraubenfeder umfasst.

11. Gefalteter absorbierender Gegenstand nach einem der vorstehenden Ansprüche, bei dem das Abstandselement (620) eine Blattfeder umfasst.

12. Gefalteter absorbierender Gegenstand nach einem der vorstehenden Ansprüche, bei dem das Abstandselement (120) ein längliches elastisches Element umfasst, das mit einer Vorspannung an dem Deckelabschnitt (411) angebracht ist.

13. Gefalteter absorbierender Gegenstand nach einem der vorstehenden Ansprüche, bei dem das Abstandselement (120) einen ersten inaktiven Zustand und einen zweiten aktiven Zustand aufweist.

14. Gefalteter absorbierender Gegenstand nach Anspruch 13, bei dem das Abstandselement (120) aus dem inaktiven Zustand in den aktiven Zustand gebracht werden kann, indem das Abstandselement (120) gehandhabt wird.

15. Gefalteter absorbierender Gegenstand nach einem der vorstehenden Ansprüche, bei dem das Abstandselement (120) ein lösbares Siegelungselement (121) für den Deckelabschnitt (111) der Verpackungshülle (100) umfasst.

16. Gefalteter absorbierender Gegenstand nach Anspruch 15, bei dem Siegelungselement (121) ein wiederverschließbares Siegelungselement (121) ist.

## Revendications

1. Article absorbant plié (101) destiné à l'absorption de fluides corporels et conditionné dans un emballage de conditionnement (100), dans lequel l'emballage de conditionnement (100) comporte une partie de conteneur (112) ayant un intérieur et un extérieur et une ouverture (118) pour retirer l'article absorbant et une partie de couvercle (111) ayant une position ouverte et une position fermée, dans lequel la partie de couvercle (111) dans la position fermée ferme l'ouverture (118) de la partie de conteneur (112) et est reliée de manière libérable à l'extérieur de la partie de conteneur (112), **caractérisé en ce qu'**un élément d'écartement (120) est agencé entre la partie de couvercle (111) et la partie de conteneur (112), l'élément d'écartement ayant la capacité de créer une distance d'au moins 0,5 mm entre la partie de couvercle et la partie de conteneur et constituant un moyen pour faciliter l'ouverture de l'emballage de conditionnement (100).

2. Article absorbant plié selon la revendication 1, dans lequel la partie de couvercle (111) a une première extrémité située au niveau de l'ouverture (118) de la partie de conteneur et une seconde extrémité ayant un bord d'extrémité (105), l'élément d'écartement (120) étant positionné près du bord d'extrémité (105).

3. Article absorbant plié selon la revendication 1 ou 2, dans lequel l'emballage de conditionnement (100) est formé à partir d'une pièce rectangulaire de matériau (113) ayant une direction longitudinale et une direction transversale et ayant deux bords latéraux (102, 103) s'étendant dans la direction longitudinale et deux bords d'extrémité (104, 105) s'étendant dans la direction transversale et dans lequel l'emballage de conditionnement (100) comporte deux lignes de pli (106, 107) agencées dans la direction transversale et divisant l'emballage de conditionnement (100) en un premier panneau d'extrémité (108), un second panneau d'extrémité (110) et un panneau central (109), le second panneau d'extrémité (110) et le panneau central (109) étant reliés l'un à l'autre dans des jonctions de bord latéral (114, 115) le long des bords latéraux (102, 103) formant ainsi la partie de conteneur (112) de l'emballage de conditionnement (100) et le premier panneau d'extrémité (108) formant la partie de couvercle (111) de l'emballage de conditionnement (100).

4. Article absorbant plié selon la revendication 3, dans lequel la partie de couvercle (111) est reliée aux jonctions de bord latéral (114, 115) à l'aide d'une fixation déchirable.

5. Article absorbant plié selon la revendication 4, dans lequel la fixation déchirable est constituée d'une jonction soudée déchirable.

6. Article absorbant plié selon l'une quelconque des revendications précédentes, dans lequel l'élément d'écartement (120) a la capacité de créer une distance d'au moins 0,5 mm entre la partie de couvercle (111) et la partie de conteneur (112).

7. Article absorbant plié selon l'une quelconque des revendications précédentes, dans lequel l'élément d'écartement (120 ; 920 ; 1020) comprend d'un matériau élastiquement compressible.

8. Article absorbant plié selon l'une quelconque des revendications précédentes, dans lequel l'élément d'écartement (120) comprend d'un matériau de mousse à cellules ouvertes.

9. Article absorbant plié selon l'une quelconque des revendications précédentes, dans lequel l'élément d'écartement (120) comprend une ouate fibreuse.

10. Article absorbant plié selon l'une quelconque des revendications précédentes, dans lequel l'élément d'écartement (920) comprend un ressort hélicoïdal.

11. Article absorbant plié selon l'une quelconque des revendications précédentes, dans lequel l'élément d'écartement (620) comprend un ressort à lame.

12. Article absorbant plié selon l'une quelconque des revendications précédentes, dans lequel l'élément d'écartement (120) comprend un élément élastique allongé qui est fixé avec pré-étirement sur la partie de couvercle (411).

13. Article absorbant plié selon l'une quelconque des revendications précédentes, dans lequel l'élément d'écartement (120) a un premier état inactif et un second état actif.

14. Article absorbant plié selon la revendication 13, dans lequel l'élément d'écartement (120) peut être amené depuis l'état inactif jusqu'à l'état actif par manipulation de l'élément d'écartement (120).

15. Article absorbant plié selon l'une quelconque des revendications précédentes, dans lequel l'élément d'écartement (120) comprend un élément de fermeture libérable (121) pour la partie de couvercle (111) de l'emballage de conditionnement (100).

16. Article absorbant plié selon la revendication 15, dans lequel l'élément de fermeture (121) est un élément de fermeture refermable (121).
